# EUROPÄISCHE PATENTSCHRIFT

(11) **EP 2 424 458 B1**
(45) Veröffentlichungstag und Bekanntmachung des Hinweises auf die Patenterteilung: **05.06.2019**
(21) Anmeldenummer: 10713140.1
(22) Anmeldetag: 07.04.2010
(51) Int. Cl.: A61B 18/12

(54) **HF-CHIRURGIEGENERATOR UND VERFAHREN ZUM BETREIBEN EINES HF-CHIRURGIEGENERATORS**
HF SURGERY GENERATOR AND METHOD FOR OPERATING AN HF SURGERY GENERATOR
GÉNÉRATEUR CHIRURGICAL HF, ET PROCÉDÉ PERMETTANT DE FAIRE FONCTIONNER UN GÉNÉRATEUR CHIRURGICAL HF

(30) Priorität: 29.04.2009 DE 102009019373; 17.08.2009 DE 102009037693
(43) Veröffentlichungstag der Anmeldung: 07.03.2012
(73) Patentinhaber: Erbe Elektromedizin GmbH, 72072 Tübingen (DE)
(72) Erfinder: SCHALL, Heiko, 72622 Nürtingen (DE); FRITZ, Martin, 72070 Tübingen (DE)
(74) Vertreter: Rüger Abel
(86) Internationale Anmeldenummer: PCT/EP2010/002186
(87) Internationale Veröffentlichungsnummer: WO 2010/124785

(56) Entgegenhaltungen:
- WO-A1-98/07378
- WO-A1-98/27880
- WO-A1-03/090635
- DE-A1- 10 046 592
- US-A- 4 281 373
- US-B1- 6 261 286

## Beschreibung

Die Erfindung betrifft einen HF-Chirurgiegenerator nach dem Patentanspruch 1 sowie ein Verfahren zum Betreiben eines solchen.

HF-Chirurgiegeneratoren werden in zunehmendem Maße zum Schneiden und zum Koagulieren von Gewebe verwendet. Einer der Vorteile von HF-chirurgischen Schnitten im Vergleich zu mechanischen Schnitten ist in der Hämostase der Schnittränder zu sehen, die insbesondere durch thermische Koagulation zustande kommt. Die Tiefe der Koagulationszone ist hierbei weitgehend abhängig von der Durchblutung des behandelten Gewebes, woraus die Anforderung an die HF-Chirurgiegeneratoren resultiert, die Tiefe der Koagulationszone möglichst reproduzierbar einstellen zu können. Insbesondere kommt es hierbei auf die Form des HF-Ausgangssignals des HF-Chirurgiegenerators an und zwar insbesondere auf das Verhältnis von Spitzenwert zu Effektivwert. Darum werden amplitudenmodulierte HF-Spannungen (-Ströme) gewählt, wobei die Modulation so weit geht, dass der HF-Chirurgiegenerator nach Möglichkeit auch nur eine einzige Schwingungsperiode - gefolgt von einer längeren Pause - erzeugen soll.

Eine weitere Forderung besteht darin, dass eine möglichst hohe Effizienz des HF- Chirurgiegenerators sichergestellt wird, da bei einem niedrigen Wirkungsgrad Wärmeverluste durch aufwändige Kühlmaßnahmen beseitigt werden müssen, was im Operationssaal nicht gewünscht ist.

Aus der DE 100 46 592 A1 ist ein HF-Chirurgiegenerator bekannt, der einen höheren Wirkungsgrad dadurch bekommen soll, dass die bei derartigen HF- Chirurgiegeneratoren üblicherweise vorgesehene Gleichspannungsversorgung in zwei Betriebsarten arbeiten kann und zwar einerseits (konventionell) zur Versorgung des Leistungsoszillators und andererseits in einem Betrieb, in welchem ein Energietransfer von der Versorgung des Leistungsoszillators zurück zum Eingang der Gleichspannungsversorgung erfolgt. Wesentliche Verbesserungen bei der Formung kurzer Pulse sind hier nicht erzielbar.

Aus der DE 100 46 592 A1 ist ein HF-Chirurgiegenerator bekannt. Bei diesem Generator wird dann, wenn das HF- Ausgangssignal beendet werden soll, der Ausgangsschaltung Energie durch einen Ohm'schen Widerstand entzogen, der sozusagen parallel zum Verbraucher geschaltet wird. Der Wirkungsgrad dieses Gerätes ist gering.

Aus der WO 98/27880 A1 ist ein Hochfrequenzgenerator zum Einsatz in der Elektrochirurgie bekannt, wobei dieser Hochfrequenzgenerator einen von einer Halbbrücke gespeisten Schwingkreis enthält. Den beiden miteinander in Reihe geschalteten Transistoren der Halbbrücke sind jeweils Freilaufdioden parallel geschaltet, die darauf eingerichtet sind, Rückwärtsströme von den Transistoren der Halbbrücke fernzuhalten. Solche Rückwärtsströme fließen, solange die von dem angeschlossenen Schwingkreis ankommenden Spannungen groß genug sind, auf die Versorgungsleitung zurück.

Aus der US 6,261,286 B1 ist ein Generator bekannt, bei dem zwischen der Primärspule eines in einem Schwingkreis eingebundenen Transformators und dem elektronischen Schalter die Primärspule eines weiteren Transformators vorgesehen ist, dessen Sekundärwicklung über eine Einweggleichrichtung mit der Speisespannung verbunden ist. Dies dient insbesondere der Abfuhr von Energie, die durch den primärseitig fließenden Strom der Transformatoren gespeichert ist.

Weiter ist aus der WO 98/07378 A1 ein elektrochirurgischer Generator bekannt, der zur Zwischenspeicherung von Restenergie aus einer Schwingspule einen gesonderten Kondensator aufweist, der über einen ihm zugeordneten Aktivierungsschalter aktiviert werden kann.
Der Erfindung liegt die Aufgabe zu Grunde, einen HF-Chirurgiegenerator der eingangs genannten Art sowie ein Verfahren zum Betreiben eines solchen derart aufzuzeigen, dass bei einem hohen Wirkungsgrad dennoch eine exakte und schnelle Formung des HF-Ausgangssignals erzielbar ist.
Diese Aufgabe wird durch einen HF-Chirurgiegenerator nach Anspruch 1 und durch ein Verfahren zum Betreiben eines solchen nach Anspruch 12 gelöst.
Insbesondere wird die Aufgabe durch einen HF-Chirurgiegenerator gelöst, umfassend ein Netzteil mit mindestens einem Speicherkondensator, eine steuerbare Schalteinrichtung mit mindestens einem Energiespeicher, z.B. einem Ausgangstransformator, über welche ein einem HF-Chirurgieinstrument zuführbares HF-Ausgangssignal erzeugt wird, wobei eine Rückspeiseeinrichtung zwischen dem Energiespeicher und dem Speicherkondensator geschaltet ist und wobei eine Steuerung vorgesehen ist, welche die Schalteinrichtung und die Rückspeiseeinrichtung derart steuert, dass dann, wenn das HF-Ausgangssignal beendet werden soll, die Rückspeiseeinrichtung mindestens zeitweise stromdurchlässig wird und der Energiespeicher in den Speicherkondensator mindestens teilweise entladen wird.

Ein wesentlicher Punkt der Erfindung liegt also darin, dass die in den üblicherweise vorgesehenen Blindelementen, z.B. dem üblicherweise vorgesehenen Ausgangstransformator, gespeicherte elektrische bzw. auch magnetische Energie diesem Energiespeicher entzogen wird, wodurch das HF-Ausgangssignal schnell auf Null zurück geführt wird. Diese Energie, die dem Speicherkondensator des Netzteils zugeführt wird, steht dann für ein nächstes HF-Ausgangssignal zur Verfügung. Dies erhöht den Wirkungsgrad der Anordnung. Eine Verbesserung der Signalsteuerbarkeit bei gleichzeitig geringem Schaltungsaufwand ist dann gegeben, wenn eine Entladeeinrichtung vorgesehen ist, welche Restenergie aus dem Energiespeicher abführt. Hierfür wird auch eine Umsetzung in Wärme durch eine Ohm'sche Last verwendet, wodurch ein zuverlässiges Ausschwingen des Oszillators gewährleistet wird. Dennoch bleibt eine erhebliche Steigerung des Wirkungsgrades durch die Rückspeisung übrig. Erfindungsgemäß wird also die Entladeeinrichtung von der Steuerung derart gesteuert, dass das Abführen der Restenergie nach einem teilweisen Entladen des Energiespeichers durchgeführt wird.

Bei einer bevorzugten Ausführungsform umfasst der Energiespeicher einen Ausgangstransformator und die Rückspeiseeinrichtung umfasst eine gesonderte Wicklung auf dem Ausgangstransformator. Auf diese Weise sind die gewünschten Spannungsverhältnisse leicht einstellbar.

Es ist möglich, die Rückspeisung über einen elektronischen Schalter zu bewerkstelligen, der von der Steuerung gesteuert wird. Alternativ ist eine Rückspeisung über eine Diodenstrecke möglich, über welche der Energiespeicher bei Beendigung des HF-Ausgangssignals entladen wird.

Der Speicherkondensator des HF-Chirurgiegenerators ist vorzugsweise in der Weise angeordnet, dass er - unter Zwischenschaltung von steuernden Elementen - die überschüssige Energie des Ausgangstransformators aufnehmen kann.

Die Rückspeiseeinrichtung umfasst einen DC/DC-Wandler, um eine entsprechende Spannungsanpassung vornehmen zu können. Dieser kann als Hochsetzsteller ausgebildet sein.

Bei einer alternativen Ausführungsform umfasst die Rückspeiseeinrichtung einen Tiefsetzsteller, dessen Eingangsspannung mittels eines elektronischen Schalters auf eine Konstantspannung geregelt wird. Diese Ausbildung der Schaltung ist besonders einfach.

Das Verfahren zum Betreiben eines HF-Chirurgiegenerators, der ein Netzteil mit mindestens einem Speicherkondensator und eine steuerbare Schalteinrichtung und einen Ausgangstransformator aufweist, der ein HF-Ausgangssignal erzeugt, das einem HF-Chirurgieinstrument zuführbar ist, zeichnet sich dadurch aus, dass zum Abschalten des HF-Ausgangssignals im Ausgangstransformator gespeicherte Energie dem Speicherkondensator zugeführt und in diesem gespeichert wird. Die Vorteile dieses Verfahrens wurden oben bereits erläutert. Gleiches gilt auch für entsprechende Weiterbildungen des Verfahrens, die anhand der oben beschriebenen Schaltungsmerkmale erkannt werden können.

Nachfolgend werden Ausführungsbeispiele der Erfindung anhand von Abbildungen näher erläutert. Hierbei zeigen
- Fig. 1 ein Schaltbild einer ersten Ausführungsform der Erfindung;
- Fig. 2 ein Schaltbild einer zweiten Ausführungsform der Erfindung;
- Fig. 3 ein Schaltbild einer dritten Ausführungsform der Erfindung;
- Fig. 4 ein Schaltbild einer vierten Ausführungsform der Erfindung;
- Fig. 5 ein Zeitdiagramm von Signalabläufen; und
- Fig. 6 ein Schaltbild einer fünften Ausführungsform der Erfindung.

In der nachfolgenden Beschreibung werden für gleiche oder gleich wirkende Teile dieselben Bezeichnungen verwendet.

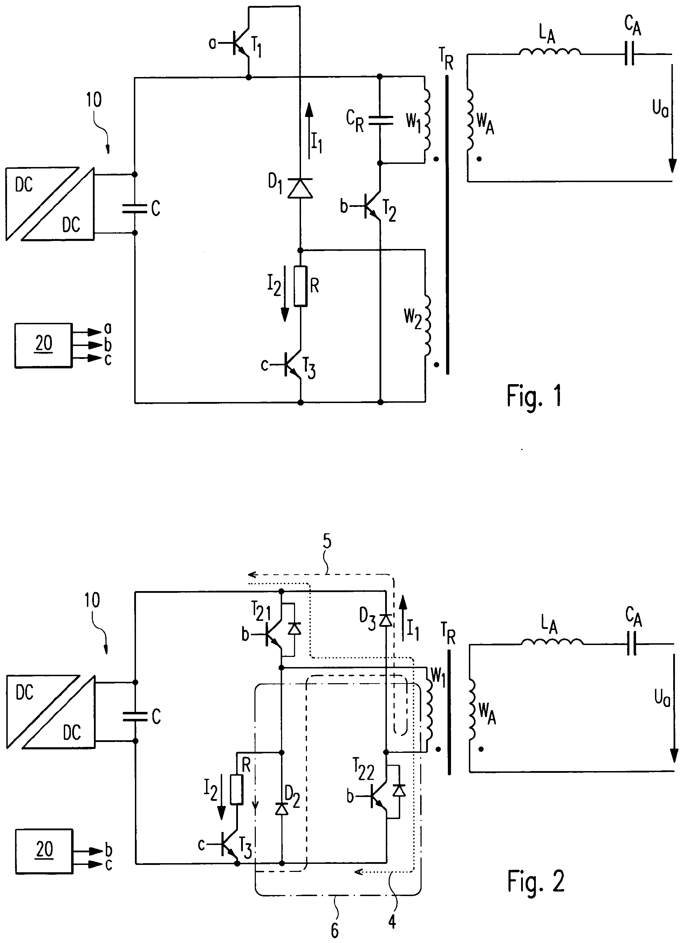

Bei der in Fig. 1 gezeigten Schaltung ist mit der Bezugsziffer 10 ein Netzteil bezeichnet, von welchem lediglich der DC/DC-Wandler mit einem am Ausgang liegenden Kondensator C bezeichnet ist. Selbstverständlich sind weitere Wandlereinrichtungen vorgesehen, um den DC/DC-Wandler aus dem Wechselstromnetz zu speisen.

Der Generator umfasst einen Schwingkreis C_{R} und eine (Primär-) Wicklung W₁ eines Ausgangstransformators T_{R} und ist über einen Transistor T₂ mit dem Kondensator C verbunden. Ein Steuerungseingang b des Transistors T₂ ist mit einer Steuerung 20 verbunden, die somit durch eine entsprechende Ansteuerung des Transistors T₂ im Schwingkreis C_{R}-W₁ eine Schwingung erzeugen kann, die über eine Sekundärspule W_{A} und einen Reihenschwingkreis L_{A}/ C_{A} als Ausgangsspannung Uₐ am HF-chirurgischen Instrument (nicht gezeigt) übergeben werden kann.

Eine weitere Wicklung W₂ des Transformators T_{R} ist über eine Diode D₁ und einen Transistor T₁ einerseits und über eine direkte Leitung andererseits ebenfalls mit dem Kondensator C verbunden. Der Transistor T₁ ist mit seinem Steuereingang a mit der Steuerung 20 verbunden. Parallel zur weiteren Wicklung W₂ befindet sich eine Reihenschaltung aus einem Widerstand R und einem Transistor T₃, dessen Steuereingang c ebenfalls mit der Steuerung 20 verbunden ist.

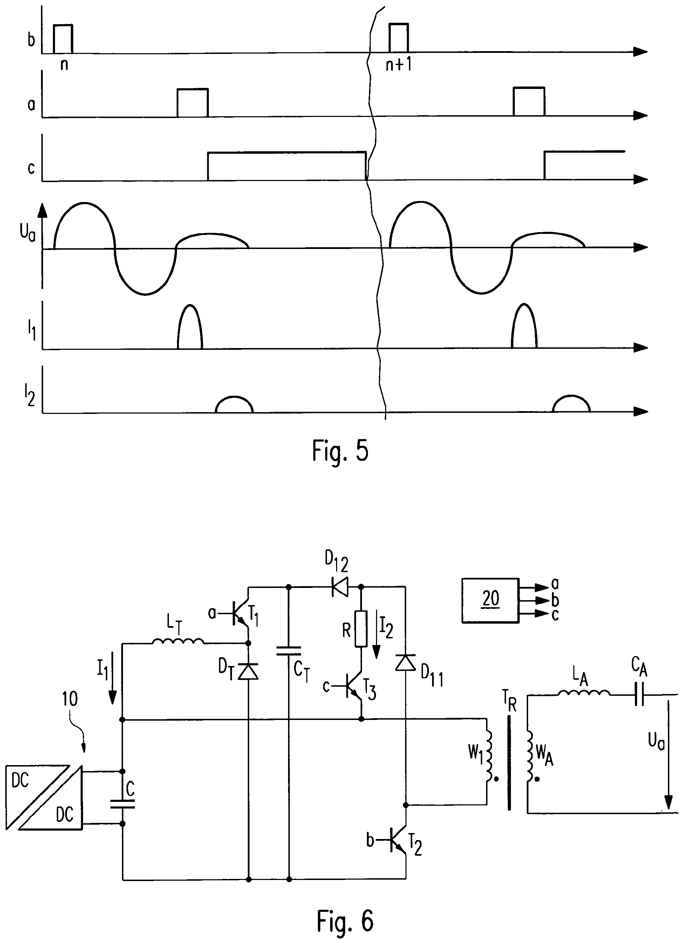

Nachfolgend wird die Funktionsweise der Schaltung nach Fig. 1 anhand von Fig. 5 erläutert.

Die Steuerung 20 erzeugt Ansteuerimpulse n und n+1, die dem Steuereingang b des Transistors T₂ übermittelt werden. Durch die Ansteuerung des Transistors T₂ wird der Schwingkreis C_{R}; W₁ erregt und erzeugt eine Ausgangsspannung Uₐ.

Um die Schwingung des Schwingkreises C_{R}, W₁ zu beenden, erzeugt die Steuerung 20 ein Signal a, das den Transistor T₁ durchsteuert. Demzufolge ist nur die weitere Wicklung W₂ über die Diode D₁ und den Transistor T₁ mit dem Kondensator C verbunden, so dass ein Strom I₁ fließt, der die im Transformator T_{R} gespeicherte Energie in den Kondensator C überführt.

Nach dem relativ kurzen Steuerimpuls a (siehe Fig. 5) wird der Transistor T₃ von der Steuerung 20 über seinen Steuereingang c durchgeschaltet, so dass nun ein Strom I₂ durch den Widerstand R fließt, wodurch eine Restentladung der Energie aus dem Transformator T_{R} in Wärme umgesetzt wird. Die Ausgangsspannung Uₐ wird somit vollständig auf Null gesetzt, wobei ein wesentlicher Teil der im Ausgangstransformator T_{R} gespeicherten Energie am Kondensator C wieder zur Verfügung steht, um bei einem nächsten Durchsteuern des Transistors T₂ über einen Steuerimpuls b (Impuls n+1) wieder verwendet zu werden.

Die Schaltung gemäß Fig. 2 ist hinsichtlich des Netzteils 10 und der Ausgangsschaltung ebenso aufgebaut wie die Schaltung nach Fig. 1. Bei dieser Schaltung steht die Primärwicklung W₁ des Transformators T_{R} über einen Transistor T₂₁ mit einem oberen Pol des Kondensators C und über einen Transistor T₂₂ mit einem unteren Pol des Kondensators C in Verbindung. Die Kollektor-Emitter-Strecken der beiden Transistoren T₂₁ und T₂₂ sind durch Freilaufdioden überbrückt.

Der Kollektor des unteren Transistors T₂₂ ist über eine Diode D₃ mit dem oberen Pol des Kondensators C verbunden. Der Emitter des Transistors T₂₁ ist über eine Diode D₂ mit dem unteren Pol des Kondensators C verbunden, wobei die Diode D₂ über eine Reihenschaltung aus einem Widerstand R und einem Transistor T₃ überbrückt ist.

Zur Erläuterung der Funktion dieser Schaltung nach Fig. 2 sind die Strompfade angegeben.

Wenn der Transistor T₂₁ und der Transistor T₂₂ durchgesteuert werden, so ergibt sich der Strompfad 4, der eine (pulsförmige) Ausgangsspannung Uₐ erzeugt. Um diesen Ausgangspuls schnell zu beenden, wird (nach Schließen der Transistoren T₂₁, T₂₂) die im Transformator T_{R} enthaltene Energie als Strom I₁ über die Dioden D₂ und D₃ auf den Kondensator C zurückgeführt. Um eine Restenergie abzubauen, steuert die Steuerung 20 den Transistor T₃ an, so dass die im Transformator T_{R} enthaltene Restenergie als Strom I₂ über den Strompfad 6 in Wärme umgesetzt wird.

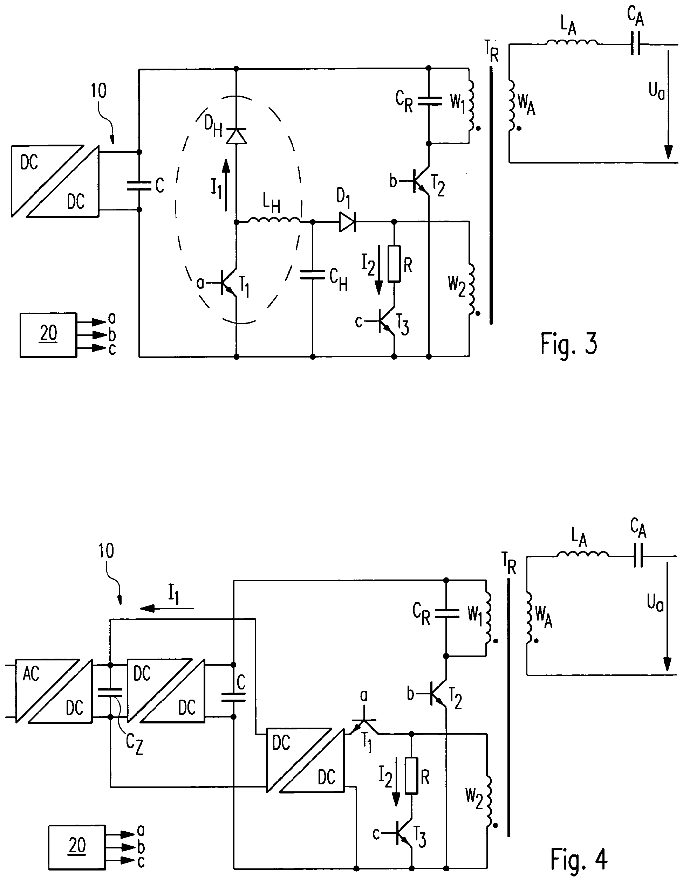

Die in Fig. 3 gezeigte Anordnung ähnelt hinsichtlich der Schwingungserzeugung und hinsichtlich des Transformators T_{R} der Schaltung nach Fig. 1. Jedoch ist bei dieser Anordnung die zusätzliche Wicklung W₂ über einen Hochsetzsteller, umfassend eine Diode D₁, einen Kondensator C_{H}, eine Induktivität L_{H} und eine weitere Diode D_{H} mit dem Kondensator C verbunden. Genauer gesagt, liegt parallel zur zusätzlichen Wicklung W₂ eine Reihenschaltung, gebildet aus der Diode D₁ und Kondensator C_{H}. Am Kopplungspunkt zwischen der Diode und dem Kondensator liegt ein Ende der Spule L_{H}, deren anderes Ende über die Diode D_{H} mit dem Kondensator C verbunden ist. Der Kopplungspunkt zwischen der Diode D_{H} und der Induktivität L_{H} ist über einen Transistor T₁ mit dem anderen Pol des Kondensators C verbunden.

Weiterhin ist parallel zur zusätzlichen Wicklung W₂ eine Reihenschaltung bestehend aus einem Ohm'schen Widerstand R und einem Transistor T₃ geschaltet.

Die Ansteuerung erfolgt gemäß Diagramm nach Fig. 5, wobei jedoch der Rückspeisetransistor T₁ auch - im Unterschied zur Darstellung in Fig. 5 - wie folgt angesteuert werden kann: Die hier gezeigte Schaltung bestehend aus L_{H}, T₁ und D_{H} stellt einen Hochsetzsteller dar. Dieser Hochsetzsteller vermag Energie vom Speicherkondensator C_{H} auf den Eingangskondensator C umzuladen. Die Ansteuerung des Transistors T₁ kann in Pulsweitenmodulation (PWM), Current-Mode-Regelung oder einer ähnlichen quasikontinuierlichen Betriebsart vorgenommen werden. Das Bestreben der Regelung des Hochsetzstellers ist es, die Spannung über dem Speicherkondensator C_{H} konstant zu halten und dementsprechend den Drosselstrom durch L_{H} zu variieren.

Die in Fig. 4 gezeigte Schaltung unterscheidet sich von der nach Fig. 3 dadurch, dass nicht der in der eigentlichen Generatorschaltung vorgesehene Kondensator C sondern ein in einem Zwischenkreis vorgesehener Kondensator C_{z} zur Rückspeisung der im Transformator T_{R} gespeicherten Energie durch den Strom I₁ verwendet wird. Aus dieser Schaltung ist auch ersichtlich, dass es bei der vorliegenden Erfindung nicht auf das Element ankommt, welches die im Ausgangstransformator T_{R} gespeicherte Energie aufnimmt und zur erneuten Erregung und Erzeugung einer Ausgangsspannung Uₐ wieder zur Verfügung stellt.

Bei der in Fig. 6 gezeigten Schaltung ist zur Erregung und Erzeugung einer Ausgangsspannung Uₐ wieder ein Transistor T₂ vorgesehen, der einen Stromkreis zwischen dem Ausgangskondensator C des Netzteils 10 und der Primärwicklung W₁ des Transformators T_{R} bei Ansteuerung durch ein Signal b aus der Steuerung 20 schließt.

Zum Rückspeisen der im Transformator T_{R} enthaltenen Energie auf dem Kondensator C ist bei dieser Anordnung eine Reihenschaltung, bestehend aus einer ersten Diode D₁₁, einer zweiten Diode D₁₂, einem Transistor T₁ und einer Spule L_{T} mit dem Kondensator C verbunden. Der Verbindungspunkt zwischen dem Transistor T₁ und der Spule L_{T} ist über eine Diode D_{T} mit dem unteren Pol des Kondensators C verbunden. Der Kopplungspunkt zwischen der Diode D₁₂ und dem Transistor T₁ ist über einen Kondensator C_{T} mit dem unteren Pol des Kondensators C verbunden. Der Verbindungspunkt zwischen den Dioden D₁₂ und D₁₁ ist über eine Reihenschaltung bestehend aus einem Widerstand R und einem Transistor T₂ mit dem oberen Pol des Kondensators C verbunden.

Bei dieser Schaltung wird der für die Rückspeisung verantwortliche Transistor T₁ über seinen Steuereingang a von der Steuerung 20 in PWM-Mode (oder einem anderen kontrollierten Mode, z.B. Current-Mode oder Zweipunktregelung) betrieben und zwar derart, dass die Eingangsspannung des hier aufgebauten Tiefsetzstellers auf eine Konstantspannung zur Aufladung des Kondensators C geregelt wird. Ansonsten wird die Schaltung wie die zuvor betriebenen Schaltungen betrieben, wobei auch bei dieser Schaltung (wie bei Fig. 2) der Generator kein sinusschwingender Generator ist, sondern auf Einzelpulserregungen ausgelegt ist. Daher entfällt auch hier ein zur Primärwicklung W₁ paralleler Kondensator. Die rückgespeiste Energie beschränkt sich hier auf die im Transformator gespeicherte Magnetisierungsenergie.

## Patentansprüche

1. HF-Chirurgiegenerator, umfassend
- ein Netzteil (10) mit mindestens einem Speicherkondensator (C);
- eine steuerbare Schalteinrichtung mit mindestens einem Energiespeicher (T_{R}), z.B. einem Transformator, über welche ein, einem HF- Chirurgieinstrument zuführbares HF-Ausgangssignal (Uₐ) erzeugt wird,
wobei eine Rückspeiseeinrichtung (D₁, D₂, D₃, T₁, D_{H}, L_{H}, W₂) zwischen dem Energiespeicher (T_{R}) und dem Speicherkondensator (C) geschaltet ist, und wobei eine Steuerung (20) vorgesehen ist, welche die Schalteinrichtung (T₂) und die Rückspeiseeinrichtung (D₁, D₂, D₃, P₁, D_{H}, L_{H}, W₂) derart steuert, dass dann, wenn das HF-Ausgangssignal (Uₐ) beendet werden soll, die Rückspeiseeinrichtung (D₁, D₂, D₃, T₁, D_{H}, L_{H}, W₂) mindestens zeitweise stromdurchlässig und der Energiespeicher (T_{R}) in den Speicherkondensator (C) mindestens teilweise entladen wird,
**dadurch gekennzeichnet,**
**dass** die Steuerung (20) darauf eingerichtet ist, wenn das HF-Ausgangssignal (Uₐ) beendet werden soll, zunächst die Rückspeiseeinrichtung (D₁, D₂, D₃, T₁, D_{H}, L_{H}, W₂) zu aktivieren,
**dass** eine Entladeeinrichtung (R, T₃) vorgesehen ist, zum Abführen einer Restenergie aus dem Energiespeicher (T_{R}),
**dass** die Entladeeinrichtung eine Umsetzeinrichtung, insbesondere eine Ohm'sche Last (R), zum Umsetzen der Restenergie in Wärme umfasst, die Steuerung (20) weiter darauf eingerichtet ist, nach Aktivierung der Rückspeiseeinrichtung (D₁, D₂, D₃, T₁, D_{H}, L_{H}, W₂) die Entladeeinrichtung zu aktivieren,
wobei die Entladeeinrichtung von der Steuerung (20) derart gesteuert wird, dass das Abführen der Restenergie nach einem teilweisen Entladen des Energiespeichers (T_{R}) durchgeführt wird.

2. HF-Chirurgiegenerator nach einem der vorhergehenden Ansprüche, **dadurch gekennzeichnet, dass** der Energiespeicher einen Ausgangstransformator (T_{R}) und die Rückspeiseeinrichtung eine gesonderte Wicklung (W₁) auf dem Ausgangstransformator (T_{R}) umfassen.

3. HF-Chirurgiegenerator nach einem der vorhergehenden Ansprüche, **dadurch gekennzeichnet, dass** die Rückspeiseeinrichtung mindestens einen elektronischen Schalter (T₁) umfasst, der von der Steuerung (20) gesteuert wird.

4. HF-Chirurgiegenerator nach einem der Ansprüche 1 bis 3, **dadurch gekennzeichnet, dass** die Rückspeiseeinrichtung eine Diodenstrecke (D₂, D₃) umfasst, über welche der Energiespeicher (T_{R}) bei Beendigung des HF-Ausgangssignals (Uₐ) entladen wird.

5. HF-Chirurgiegenerator nach einem der vorhergehenden Ansprüche, **dadurch gekennzeichnet, dass** der Speicherkondensator (C) in Serie mit einem Ausgangstransformator (T_{R}) der Schalteinrichtung geschaltet ist.

6. HF-Chirurgiegenerator nach einem der Ansprüche 1 bis 5, **dadurch gekennzeichnet, dass** der Speicherkondensator (C_{z}) vor einem DC/DC-Wandler der Schalteinrichtung angeordnet ist.

7. HF-Chirurgiegenerator nach einem der vorhergehenden Ansprüche, **dadurch gekennzeichnet, dass** die Rückspeiseeinrichtung einen DC/DC-Wandler umfasst.

8. HF-Chirurgiegenerator nach einem der vorhergehenden Ansprüche, **dadurch gekennzeichnet, dass** die Rückspeiseeinrichtung einen Hochsetzsteller umfasst.

9. HF-Chirurgiegenerator nach einem der vorhergehenden Ansprüche, insbesondere nach Anspruch 7, **dadurch gekennzeichnet, dass** die Rückspeiseeinrichtung einen Tiefsetzsteller umfasst, dessen Eingangsspannung mittels eines elektronischen Schalters (T₁) auf eine Konstantspannung geregelt wird.

10. HF-Chirurgiegenerator nach Anspruch 7 oder 8, **dadurch gekennzeichnet, dass** die Rückspeiseeinrichtung in Pulsweltenmodulation angesteuert wird.

11. HF-Chirurgiegenerator nach Anspruch 7 oder 8, **dadurch gekennzeichnet, dass** die Rückspeiseeinrichtung In Current-Mode-Regelung angesteuert wird.

12. Verfahren zum Betreiben eines HF-Chirurgiegenerators, der ein Netzteil (10) mit mindestens einem Speicherkondensator (C) und eine steuerbare Schalteinrichtung mit einem Ausgangstransformator (T_{R}) aufweist, die ein HF-Ausgangssignal (Uₐ) erzeugt, das einem HF-Chirurgieinstrument zuführbar ist, wobei eine Steuerung (20) vorgesehen ist, die darauf eingerichtet ist, wenn das HF-Ausgangssignal beendet werden soll, zunächst eine Rückspeiseeinrichtung (T₁, D₁, W₂) zu aktivieren, sodass zum Abschalten des HF-Ausgangssignals im Ausgangstransformator (T_{R}) gespeicherte Energie dem Speicherkondensator (C) zugeführt und in diesem gespeichert wird, und wobei die Steuerung (20) weiter darauf eingerichtet ist, nach Aktivierung der Rückspeiseeinrichtung (T₁, D₁, W₂) eine Entladeeinrichtung (R, T3) zu aktivieren, sodass eine Restentladung der Energie aus dem Ausgangstransformator (T_{R}) in Wärme umgesetzt wird.

## Claims

1. HF surgery generator comprising:
- a power supply (10) having at least one storage capacitor (C);
- a controllable switching device having at least one energy store (T_{R}), e.g. a transformer, which generates an HF output signal (Uₐ) that can be fed to an HF surgery instrument,
wherein a feedback device (D₁, D₂, D₃, T₁, D_{H}, L_{H}, W₂) is connected between the energy store (T_{R}) and the storage capacitor (C), and wherein a controller (20) is provided to control the switching device (T₂) and the feedback device (D₁, D₂, D₃, T₁, D_{H}, L_{H}, W₂) such that when the HF output signal (Uₐ) is to be terminated, the feedback device (D₁, D₂, D₃, T₁, D_{H}, L_{H}, W₂) allows current to flow for at least part of the time and the energy store (T_{R}) in the storage capacitor (C) is at least partially discharged,
**characterised in that** the controller (20) is configured, when the HF output signal (Uₐ) is to be terminated, firstly to activate the feedback device (D₁, D₂, D₃, T₁, D_{H}, L_{H}, W₂),
that a discharge device (R, T₃) is provided for dissipating a residual energy from the energy store (T_{R}),
that the discharge device comprises a converter device, in particular an ohmic load (R), for converting the residual energy into heat,
and the controller (20) is furthermore configured to activate the discharge device after activating the feedback device (D₁, D₂, D₃, T₁, D_{H}, L_{H}, W₂),
wherein the discharge device is controlled by the controller (20) such that the residual energy is dissipated after a partial discharge of the energy store (T_{R}).

2. HF surgery generator according to any of the preceding claims, **characterised in that** the energy store comprises an output transformer (T_{R}) and the feedback device comprises a separate winding (W₁) on the output transformer (T_{R}).

3. HF surgery generator according to any of the preceding claims, **characterised in that** the feedback device comprises at least one electronic switch (T₁) which is controlled by the controller (20).

4. HF surgery generator according to any of claims 1 to 3, **characterised in that** the feedback device comprises a diode group (D₂, D₃) via which the energy store (T_{R}) is discharged on termination of the HF output signal (Uₐ).

5. HF surgery generator according to any of the preceding claims, **characterised in that** the storage capacitor (C) is connected in series with the output transformer (T_{R}) of the switching device.

6. HF surgery generator according to any of claims 1 - 5, **characterised in that** the storage capacitor (C_{z}) is arranged upstream of a DC/DC converter of the switching device.

7. HF surgery generator according to any of the preceding claims, **characterised in that** the feedback device comprises a DC/DC converter.

8. HF surgery generator according to any of the preceding claims, **characterised in that** the feedback device comprises a step-up converter.

9. HF surgery generator according to any of the preceding claims, in particular claim 7, **characterised in that** the feedback device comprises a step-down converter, the input voltage of which is regulated to a constant voltage by means of an electronic switch (T₁).

10. HF surgery generator according to claim 7 or claim 8, **characterised in that** the feedback device is actuated in pulse-width modulation.

11. HF surgery generator according to claim 7 or claim 8, **characterised in that** the feedback device is actuated in current-mode regulation.

12. Method for operating an HF surgery generator comprising a power supply (10) having at least one storage capacitor (C), and a controllable switching device having an output transformer (T_{R}) which generates an HF output signal (Uₐ) that can be fed to an HF surgery instrument, wherein a controller (20) is provided which is configured such that when the HF output signal is to be terminated, firstly it activates a feedback device (T₁, D₁, W₂) so that, to shut off the HF output signal, energy stored in the output transformer (T_{R}) is supplied to the storage condenser (C) and stored therein, and wherein the controller (20) is furthermore configured, after activation of the feedback device (T₁, D₁, W₂), to activate a discharge device (R, T₃) so that a residual discharge of energy from the output transformer (T_{R}) is converted into heat.

## Revendications

1. Générateur chirurgical HF comprenant
- un bloc d'alimentation (10) comportant au moins un condensateur de stockage (C) ;
- un dispositif de commutation commandable, comportant au moins un accumulateur d'énergie (T_{R}), par exemple un transformateur, par l'intermédiaire duquel on produit un signal de sortie (Uₐ) susceptible d'être acheminé à un instrument chirurgical HF,
un dispositif d'alimentation de retour (D₁, D₂, D₃, T₁, D_{H}, L_{H}, W₂) étant intercalé entre l'accumulateur d'énergie (T_{R}) et le condensateur de stockage (C), et une commande (20) étant prévue, qui commande le dispositif de commutation (T₂) et le dispositif d'alimentation de retour (D₁, D₂, D₃, T₁, D_{H}, L_{H}, W₂) de manière à ce que, lorsque le signal de sortie HF (Uₐ) doit être arrêté, le dispositif d'alimentation de retour (D₁, D₂, D₃, T₁, D_{H}, L_{H}, W₂) laisse au moins temporairement passer le courant, et l'accumulateur d'énergie (T_{R}) soit au moins en partie déchargé dans le condensateur de stockage (C),
**caractérisé en ce que**
la commande (20) est conçue de telle sorte que, lorsque le signal de sortie HF (Uₐ) doit être arrêté, elle active d'abord le dispositif d'alimentation de retour (D₁, D₂, D₃, T₁, D_{H}, L_{H}, W₂),
**en ce qu'**il est prévu un dispositif de décharge (R, T₃) destiné à évacuer une énergie résiduelle de l'accumulateur d'énergie (T_{R}),
**en ce que** le dispositif de décharge comprend un dispositif convertisseur, en particulier une charge ohmique (R), destiné à transformer l'énergie résiduelle en chaleur,
**en ce que** la commande (20) est en outre conçue pour activer le dispositif de décharge après l'activation du dispositif d'alimentation de retour (D₁, D₂, D₃, T₁, D_{H}, L_{H}, W₂),
le dispositif de décharge étant commandé par la commande (20) de manière telle que l'évacuation de l'énergie résiduelle s'effectue après une décharge partielle de l'accumulateur d'énergie (T_{R}).

2. Générateur chirurgical HF selon l'une des revendications précédentes, **caractérisé en ce que** l'accumulateur d'énergie comprend un transformateur de sortie (T_{R}), et le dispositif d'alimentation de retour comprend un enroulement distinct (W₁) sur le transformateur de sortie (T_{R}).

3. Générateur chirurgical HF selon l'une des revendications précédentes, **caractérisé en ce que** le dispositif d'alimentation de retour comprend au moins un commutateur électronique (T₁) qui est activé par la commande (20).

4. Générateur chirurgical HF selon l'une des revendications 1 à 3, **caractérisé en ce que** le dispositif d'alimentation de retour comprend une portion à diodes (D₂, D₃) par l'intermédiaire de laquelle l'accumulateur d'énergie (T_{R}) est déchargé lorsque le signal de sortie HF (Uₐ) est arrêté.

5. Générateur chirurgical HF selon l'une des revendications précédentes, **caractérisé en ce que** le condensateur de stockage (C) est branché en série avec un transformateur de sortie (T_{R}) du dispositif de commutation.

6. Générateur chirurgical HF selon l'une des revendications 1 à 5, **caractérisé en ce que** le condensateur de stockage (C_{z}) est disposé avant un convertisseur DC/DC du dispositif de commutation.

7. Générateur chirurgical HF selon l'une des revendications précédentes, **caractérisé en ce que** le dispositif d'alimentation de retour comprend un convertisseur DC/DC.

8. Générateur chirurgical HF selon l'une des revendications précédentes, **caractérisé en ce que** le dispositif d'alimentation de retour comprend un convertisseur élévateur.

9. Générateur chirurgical HF selon l'une des revendications précédentes, notamment selon la revendication 7, **caractérisé en ce que** le dispositif d'alimentation de retour comprend un convertisseur abaisseur dont la tension d'entrée est régulée sur une tension constante au moyen d'un commutateur électronique (T₁).

10. Générateur chirurgical HF selon la revendication 7 ou 8, **caractérisé en ce que** le dispositif d'alimentation de retour est activé en modulation d'impulsions en largeur.

11. Générateur chirurgical HF selon la revendication 7 ou 8, **caractérisé en ce que** le dispositif d'alimentation de retour est activé en régulation à mode de courant.

12. Procédé de fonctionnement d'un générateur chirurgical HF qui comprend un bloc d'alimentation (10), comportant au moins un condensateur de stockage (C), et un dispositif de commutation commandable, comportant un transformateur de sortie (T_{R}), qui produit un signal de sortie (Uₐ) susceptible d'être acheminé à un instrument chirurgical HF, sachant qu'il est prévu une commande (20) qui est conçue de telle sorte que, lorsque le signal de sortie HF doit être arrêté, elle active d'abord un dispositif d'alimentation de retour (T₁, D₁, W₂),
de manière à ce que, pour arrêter le signal de sortie HF, de l'énergie stockée dans le transformateur de sortie (T_{R}) soit acheminée au condensateur de stockage (C) et soit stockée dans celui-ci, et sachant que la commande (20) est par ailleurs conçue pour activer un dispositif de décharge (R, T3), après l'activation du dispositif d'alimentation de retour (T₁, D₁, W₂), de façon à ce qu'une décharge résiduelle de l'énergie du transformateur de sortie (T_{R}) soit transformée en chaleur.
